**Europäisches Patentamt**

(19) **European Patent Office**

**Office européen des brevets**

(11) Publication number: **0 145 174**
**B1**

(12) # EUROPEAN PATENT SPECIFICATION

(45) Date of publication of patent specification: **13.09.89**

(21) Application number: **84307098.8**

(22) Date of filing: **16.10.84**

(51) Int. Cl.⁴: **C 07 K 13/00,** C 07 K 15/26, C 07 H 21/04, C 12 N 15/00, C 12 P 21/02, A 61 K 45/02 // C12R1:19

(54) Novel, physiologically active polypeptides and production method thereof.

(30) Priority: **17.10.83 JP 194539/83**

(43) Date of publication of application:
**19.06.85 Bulletin 85/25**

(45) Publication of the grant of the patent:
**13.09.89 Bulletin 89/37**

(84) Designated Contracting States:
**AT BE CH DE FR GB IT LI LU NL SE**

(56) References cited:
EP-A-0 077 670
EP-A-0 083 777
EP-A-0 088 540
EP-A-0 089 676

(73) Proprietor: **SUNTORY KABUSHIKI KAISHA, also known as SUNTORY LTD.**
1-40, Dojimahama 2-chome
Kita-ku Osaka530 (JP)

(72) Inventor: **Tanaka, Shoji**
8-46, Minamikaneden-cho 1-chome
Suita Osaka 564 (JP)
Inventor: **Nomura, Midori**
20-31, Habikino 4-chome
Habikino Osaka 583 (JP)

(74) Representative: **Burford, Anthony Frederick et al**
W.H. Beck, Greener & Co. 7 Stone Buildings
Lincoln's Inn
London WC2A 3SZ (GB)

Courier Press, Leamington Spa, England.

## Description

This invention relates to novel, physiologically active polypeptides and a method of producing the same.

The physiologically active polypeptides according to the invention carry the amino acid sequence contained in mature human gamma interferon (also called immune interferon) but are novel polypeptides differing therefrom in physico-chemical properties and having very high antiviral and antiproliferative activities as compared therewith. The invention also relates to a method of producing said novel polypeptides using recombinant DNA technology as well as cultures containing said polypeptides.

Interferons are classified into three types, namely types α, β and γ, depending on their physico-chemical properties and immunological properties, among others. All interferons are substances each consisting of a polypeptide and having an antiviral activity. Among them, γ-type interferon (also called immune interferon) has potent cell growth inhibiting activity and therefore the use thereof as an antitumor or immunoregulating agent as well as the use as an antiviral agent has attracted much attention. As far as human gamma interferon (hereinafter abbreviated as "hIFN-γ") is concerned, its amino acid sequence was shown at the Second Annual International Congress for Interferon Research held at San Francisco, USA in October, 1981. Later, the DNA sequence of the human gene coding for said amino acid sequence was also revealed (R. Devos et al., Nucl. Acids Res., 10: 2487, 1982 and P. W. Gray et al., Nature, 295: 503, 1982). Furthermore a patent application claiming a method of producing pre-hIFN-γ and mature hIFN-γ using a cDNA prepared based on a human lymphocyte-derived mRNA has been laid open (Japanese Kokai Tokkyo Koho No. 58-90514).

Natural hIFN-γ derived from human lymphocytes or spleen cells is a glycoprotein. Its physico-chemical characteristics, however, are not yet clear in many respects. The protein portion of mature hIFN-γ has been shown, by using the recombinant DNA technology, to be a polypeptide composed of 146 amino acids. Nevertheless, the true structure of the active site or C-terminal site (e.g. whether the C-terminal amino acid is in the free form or in some or other modified form) is yet unknown.

As a reslt of their intensive study of the effects of the active site and C-terminal site of hIFN-γ consisting of 1460 amino acids (hereinafter abbreviated as "GIF146") on the physiological activities, the present inventors found that the polypeptide portion from the first to the 135th amino acid (hereinafter abbreviated as "GIF135"), in particular, is much superior in physiological activity to GIF146 and differs also in physico-chemical properties therefrom and that said GIF135 polypeptide can be produced by recombinant DNA technology using a chemically synthesized gene. The above surprising findings have now led to the present invention. The polypeptides of the present invention as represented by GIF135 can be extracted from cultivated transformant cells more easily as compared with GIF145 and moreover are characterized by higher stability. Therefore, they can be regarded as novel, physiologically active substances different from the prior art GIF146.

Thus, the invention provides novel, physiologically active polypeptides represented by the amino acid sequence:

```
        1                           10
  X Cys Tyr Cys Gln Asp Pro Tyr Val Lys Glu
                                        20
    Ala Glu Asn Leu Lys Lys Tyr Phe Asn Ala
                                        30
    Gly His Ser Asp Val Ala Asp Asn Gly Thr
                                        40
    Leu Phe Leu Gly Ile Leu Lys Asn Trp Lys
                                        50
    Glu Glu Ser Asp Arg Lys Ile Met Gln Ser
                                        60
    Gln Ile Val Ser Phe Tyr Phe Lys Leu Phe
                                        70
    Lys Asn Phe Lys Asp Asp Gln Ser Ile Gln
                                        80
    Lys Ser Val Glu Thr Ile Lys Glu Asp Met
                                        90
    Asn Val Lys Phe Phe Asn Ser Asn Lys Lys
                                       100
    Lys Arg Asp Asp Phe Glu Lys Leu Thr Asn
    Tyr Ser Val Thr Asp Leu Asn Val Gln Arg
    Lys Ala Ile His Glu Leu Ile Gln Val Met
    Ala Glu Leu Ser Pro Ala Ala Lys Thr Gly
    Lys Arg Lys Arg Ser
```

wherein X is a hydrogen atom or a methionyl group. The invention includes DNA sequences coding for such polypeptides, expression vectors in which any of said sequences is connected with a DNA sequence or sequences capable of causing efficient expression of the above polypeptides, single cell microorganism transformants obtained by transformation with said vectors, a method of producing the above polypeptides by cultivating said transformants, and cultures of the above transformants which contain the above polypeptides.

The present invention is described below in more detail and by way of exemplification.

Referring to the drawings,

Figure 1 is a schematic representation of the process of constructing a GIF135 polypeptide expression vector, namely pYtacGIF135;

Figure 2 is a schematic representation of the process of constructing another GIF135 polypeptide expression vector, namely pINGIF135;

Figure 3 is a schematic representation of a method of modifying the base sequence corresponding to the neighborhood of the carboxyl terminal of GIF146 for the construction of a vector for GIF135 production.

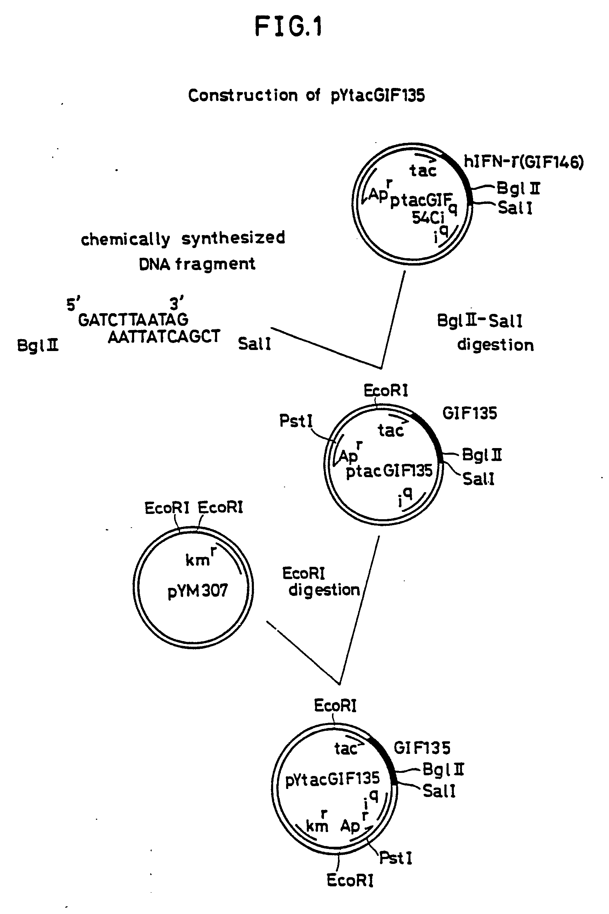

Among the GIF135-producing strains according to the invention, one (WA802/pYtacGIF135) was obtained by transforming a strain of *Escherichia coli* with a plasmid obtained by insertion of a chemically synthesized DNA fragment downstream from a chemically synthesized, GIF146-encoding gene inserted in the plasmid ptacGIF54Ci$^q$ (disclosed in Japanese Patent Application No. 58-162337) and further insertion of a runnaway plasmid DNA (cf. Fig. 1).

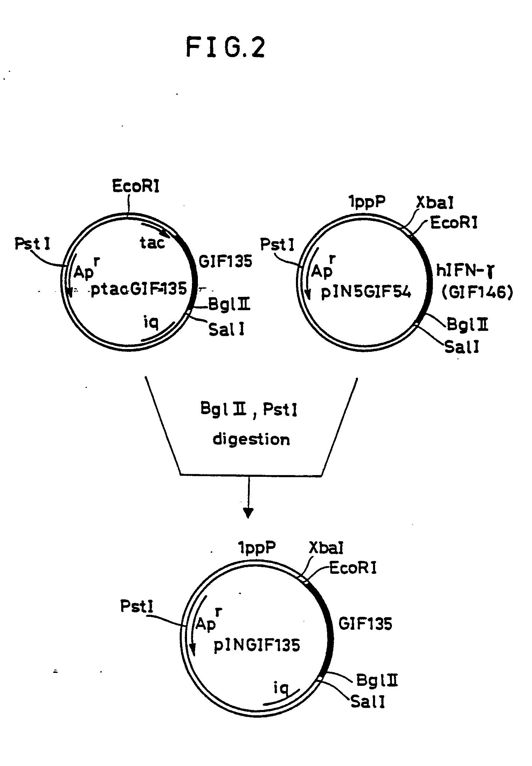

Another GIF-producing strain (WA802/pINGIF135 or W3110/pINGIF135) can be obtained by transforming the *Escherichia coli* strain WA802 or W3110 with a plasmid, pINGIF135, obtained by ligation of a *Bgl*II-*Pst*I DNA fragment (i$^q$ gene-containing fraction) from the above ptacGIF135 (Fig. 1) with a *Bgl*II-*Pst*I DNA fragment (containing the *lpp* promoter and the GIF gene) from pIN5GIF54C (disclosed in Fig. 4 of Japanese Patent Application No. 58-132524) (for said ligation, cf. Fig. 2).

WA802/pYtacGIF135 is now described in more detail. First, the plasmid ptacGIF54Ci$^q$ was treated with the restriction enzymes *Bgl*II and *Sal*I to thereby remove a DNA fragment (DNA fragment from the site coding for the 134th amino acid Arg to the *Sal*I cleavage site just downstream from the translation termination codon) occurring downstream from the GIF-encoding gene. In place of said DNA fragment, a chemically synthesized DNA fragment having the BglII and *Sal*I cohesive ends and two translation termination codons (TAA and TAG), namely

$$5'\text{GA TCT TAA TAG}^{3'}$$
$$\text{A ATT ATC AGC T}$$

was inserted, whereby the plasmid ptacGIF135 containing a GIF135-encoding gene was constructed (cf. Fig. 2). Then, the plasmid pYtacGIF135 was constructed by introducing into the plasmid constructed above the runaway DNA from pYM307 and used for transformation of *Escherichia coli* WA802. The transformant thus obtained (WA802/pYtacGIF135), when compared with the transformant (WA802/pYtacGIF) with the GIF146 gene-containing plasmid pYtacGIF with respect to antiviral activity, showed a surprisingly high antiviral activity (cf. Table 1).

pYtacGIF135 is a plasmid having the same DNA sequence as the plasmid pYtacGIF except for the downstream region of the hIFN-γ gene. The host cell used is also the same. Nevertheless, the *Escherichia coli* strain WA802/pYtacGIF135 obtained by transformation with the GIF135 gene-containing plasmid according to the invention, as the data given in Table 1 indicate, exhibited a surprisingly high antiviral activity as compared with *Escherichia coli* WA802/pYtacGIF, the transformant with pYtacGIF. Thus, cells of the transformant as obtained by growing it on L-broth containing ampicillin and kanamycin [induced culture using isopropyl β-D-thiogalactoside (IPTG)] were disrupted by freezing and thawing, followed by centrifugation. The supernatant (sup) thus obtained was about 35 times higher in antiviral activity and a fraction (ppt) obtained by extraction of the centrifugation residue with 8 M urea and 0.5 M NaCl about 96 times higher in said activity (about 73 times in total activity). Since the plasmids carried by both the transformants differ only in the hIFN-γ gene portion and the productions of the polypeptides (GIF146 and GIF135) are estimable to be theoretically equal, the results presented in Table 1 strongly suggest that GIF135 according to the invention is a polypeptide having very strong physiological activity as compared with the prior art GIF146.

The antiviral activity ratio (sup/ppt) between the supernatant (sup) obtained by cell disruption and centrifugation and the fraction (ppt) obtained by extraction of the sediment with 8 M urea and 0.5 M NaCl is 0.62 for pYtacGIF whereas the same ratio is 0.23 for pYtacGIF135. It is therefore supposed that GIF135 and GIF146 also differ in physico-chemical properties (e.g. adsorbability to the cell membrane or cell wall (cf. Table 1). Furthermore, a distinct difference in solubility was observed. For instance, GIF135 is fairly soluble in urea solutions whereas only solutions containing urea plus a salt (e.g. sodium chloride) can dissolve GIF146 to a considerable extent.

3

# EP 0 145 174 B1

## TABLE 1

### Comparison in antiviral activity

| Transformant | Antiviral activity (IU/ml) | | | sup/ppt |
|---|---|---|---|---|
| | sup[1] | ppt[2] | Total | |
| WA802/pYtacGIF | 4,700 | 7,600 | 12,300 | 0.62 |
| WA802/pYtacGIF135 | 166,600 | 728,200 | 894,700 | 0.23 |

[Notes] [1]: Supernatant fraction obtained by centrifugation following disruption of cells by freezing and thawing.

[2]: Fraction obtained by extraction of the sediment obtained in the above centrifugation with 8 M urea and 0.5 M NaCl.

The above facts indicate that the polypeptide according to the invention is not only superior in the physiological activity aspect but also provided with favorable physico-chemical properties distinct from those of GIF146. In view of the structure of GIF146, it is evidently unobvious that the polypeptide of the invention has favorable properties such as mentioned above in spite of the fact that said polypeptide is included in the polypeptide structure of GIF146.

The polypeptide of the invention is thus very interesting as a novel antiviral, antitumor or immunoregulating agent.

Analysis by electrophoresis and amino acid analysis have successfully demonstrated that the GIF135-producing strains according to the invention do produce the polypeptide consisting of 135 amino acid residues as expected. Thus, in amino acid analysis, the amino acid composition was in good agreement with the expected one and, furthermore, data suggesting that the carboxyl terminal portion of GIF135 is as expected could be obtained by carboxyl terminal analysis using carboxypeptidase.

The following examples illustrate the invention in further detail. The following examples are illustrative and the scope of the present invention is by no means limited thereto.

## Examples

### 1. Production of GIF135 .

(1) Construction of ptacGIF135

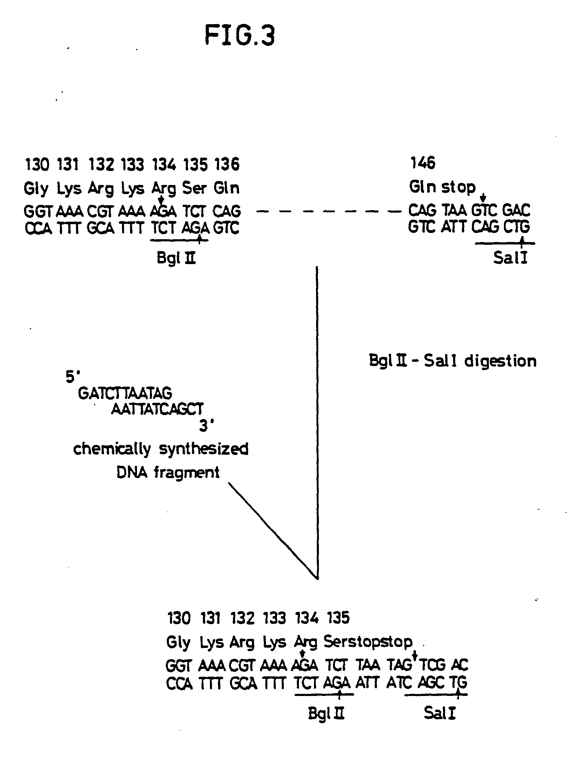

In 50 μl of 10 mM Tris-hydrochloric acid buffer (pH 7.4) containing 10 mM magnesium chloride, 150 mM sodium chloride and 10 mM 2-mercaptoethanol, there was dissolved 2 μg of the plasmid ptacGIF54Ci[q] (disclosed in Figure 3 of Japanese Patent Application No. 58-162337) DNA. To the solution were added 10 units of BglII and 10 units of SalI. After incubation at 37°C for 1 hour, the reaction mixture was subjected to agarose gel electrophoresis. The desired BglII-SalI fragment of about 7 kb was extracted electrophoretically and treated with phenol, followed by addition of alcohol, whereby the desired ca. 7 kb DNA fragment was obtained as a precipitate. Separately, 20 picomoles each of two 11-mer oligonucleotides (having the base sequences shown in Fig. 1 and Fig. 3, respectively) synthesized chemically by the solid phase method (cf. Japanese Patent Application No. 57-86180) were dissolved in 10 μl of 66 mM Tris-hydrochloric acid buffer (pH 7.6) containing 6.6 mM magnesium chloride. To the solution were added 1 μl of 4 mM ATP and 2 units of polynucleotide kinase, followed by incubation at 37°C for 30 minutes for 5′-OH phosphorylation. The approximately 7 kb BglII-SalI DNA fragment previously prepared was dissolved in 10 μl of 66 mM Tris buffer (pH 7.6) containing 6.6 mM magnesium chloride (hereinafter referred to as "ligation solution"), 10 μl of the chemically synthesized DNA fragment solution was added thereto and, following addition of 1 unit of T4 DNA ligase, the ligation reaction was effected at 16°C overnight. Using the resultant DNA, Escherichia coli WA802 was transformed in the conventional manner. The transformant selection was conducted using a nutrient agar medium containing 40 μg/ml of ampicillin and plasmid DNAs were isolated from 18 transformants out of a number ampicillin-resistant transformants obtained and analyzed using restriction enzymes. All the plasmids were found to be the contemplated ptacGIF135.

(2) Construction of pYtacGIF135

For the purpose of achieving increased expression of GIF135 through an increase in copy number, the longer EcoRI DNA fragment from the runaway plasmid pYM307 was inserted into ptacGIF135 at the EcoRI

4

cleavage site thereof. The pYM307-carrying strain of *Escherichia coli* was named W620recA/pYM307. Said strain has been deposited with the Fermentation Research Institute, Agency of Industrial Science and Technology under the deposit number FERM BP-345.

First, 2 µg of ptacGIF135 DNA and 2 µg of pYM307 DNA were respectively dissolved in 50 µl of 33 mM Tris-acetic acid buffer (pH 7.6) containing 66 mM potassium acetate, 10 mM magnesium acetate and 0.5 mM dithiothretiol, followed by addition of 5 units of *Eco*RI. After incubation at 37°C for 1 hour, the restriction enzyme was inactivated by heating at 65°C for 15 minutes and then ethanol was added. The thus-obtained DNA precipitates were dried *in vacuo* and dissolved in 20 µl of ligation solution and, following addition of 1 unit of T4 DNA ligase, the ligation was carried out at 16°C overnight. The resultant DNA was used for transforming *Escherichia coli* WA802. The transformation was conducted at a temperature of 28°C. Transformants were selected on a nutrient agar medium containing 40 µg/ml of ampicillin and 20 µg/ml of kanamycin. From among a number of ampicillin- and kanamycin-resistant transformants thus obtained, 12 strains were arbitrarily chosen and subjected to analysis using restriction enzymes and, as a result, all the strains analyzed could be confirmed to carry the intended plasmid pYtacGIF135.

2. Antiviral activity measurement

For estimating the gamma interferon activity, WA802/pYtacGIF and WA802/pYtacGIF135 were respectively cultured in 2 ml of L-broth (containing 40 µg/ml of ampicillin) at 28°C overnight and 0.2 ml of the cells obtained was inoculated into 4 ml of L-broth (containing 40 µg/ml of ampicillin). After incubation at 32°C until the OD660 value reached about 0.7, 0.2 ml of 100 mM IPTG solution was added and incubation was performed at 37°C for 3 hours. A 1-ml portion of the culture broth was centrifuged at 10,000 rpm for 5 minutes. To the bacterial cells thus obtained, there was added 500 µl of 0.15 M sodium phosphate buffer (pH 7.2) containing 1 mg/ml lysozyme and 50 mM sodium chloride. The mixture was allowed to stand in an ice bath for 10 minutes. Following this bacteriolytic step, the cells were disrupted by three repetitions of rapid freezing in dry ice-ethanol and rapid thawing in a constant-temperature bath maintained at 37°C. Centrifugation at 10,000 rpm for 10 minutes gave a supernatant, which was subjected to antiviral activity measurement. The antiviral activity was measured as disclosed in Japanese Patent Application No. 57-86180. For further recovering the desired polypeptide from the precipitate fraction, 500 µl of 8 M urea solution containing 0.15 mM sodium chloride was added to the precipitate, followed by sonication and centrifugation at 10,000 rpm for 10 minutes. The supernatant thus collected was found to have a very high antiviral activity. The results of such antiviral activity measurement are shown in Table 1. The fact that, as shown in Table 1, the *Escherichia coli* strain transformed with pYtacGIF135 produced a very high antiviral activity (about 73-fold higher in total activity) as compared with the same strain transformed with pTtacGIF in spite of the use of the same expression vector by the present inventors in the above example strongly suggests that GIF135 in accordance with the invention is a polypeptide having a surpassing antiviral activity which cannot be found in the so far known GIF146.

3. Amino acid analysis

Polypeptide fractions were respectively extracted from the cultured cells of WA802/pYtacGIF135 and WA802/pYtacGIF by the method described in Example 2 and subjected to SDS gel electrophoresis. Gel fragments corresponding in molecular weight to GIF135 and GIF146 were excised, the respective polypeptides were extracted therefrom, dialyzed against water to a sufficient extent and then subjected to amino acid analysis.

The band corresponding to GIF135 on the SDS gel showed an evident shift to the lower molecular weight side from the band corresponding to GIF146 and both the bands were found at their respective expected sites (estimated on the molecular weight basis).

(1) Analysis of amino acid composition

An amount equivalent to about 1 nanomole of each polypeptide extract was evaporated to dryness under reduced pressure, 100 µl of 6 N HCl containing 0.1% phenol was added, and hydrolysis was effected under reduced pressure at 110°C for 24, 48 or 72 hours. Separately, a sample after performic acid oxidation was hydrolyzed with 6 N HCl at 110°C for 24 hours. Each hydrolyzate was subjected to amino acid analysis which was performed in the conventional manner using an amino acid analyzer (Hitachi model 835-50). The results obtained are shown in Table 2.

The values for Met (methionine) given in Table 2 suggest that both the polypeptides are not high in the content of the translation startpoint amino acid methionine if said amino acid occurs attached to the amino terminal thereof.

TABLE 2

Amino acid analysis of GIF135 and GIF146

| Amino acid | GIF135 | | GIF146 | |
|---|---|---|---|---|
| | Found | Theoretical | Found | Theoretical |
| CysSO$_3$H[A] | 1.7 | 2 | 2.3 | 2 |
| Asp | 19.8 | 20 | 19.9 | 20 |
| Thr | 5.0[B] | 5 | 5.1 | 5 |
| Ser | 10.1[B] | 10 | 10.7 | 11 |
| Glu | 16.0 | 16 | 17.7 | 18 |
| Pro | 2.2 | 2 | 2.4 | 2 |
| Gly | 4.3 | 4 | 5.2 | 5 |
| Ala | 7.2 | 7 | 8.3 | 8 |
| Val | 7.8[C] | 8 | 7.7 | 8 |
| Met | 3.0 | 3 | 3.8 | 4 |
| Ile | 6.8[C] | 7 | 6.5 | 7 |
| Leu[D] | 9.0 | 9 | 10.0 | 10 |
| Tyr | 4.5 | 5 | 4.8 | 5 |
| Phe | 8.9 | 9 | 9.6 | 10 |
| Lys | 19.5 | 20 | 20.0 | 20 |
| His | 2.0 | 2 | 1.8 | 2 |
| Arg | 5.0 | 5 | 7.7 | 8 |
| trp[E] | — | 1 | — | 1 |

Notes: [A]: Cys was calculated as CysSO$_3$H.
[B]: Calculated by extrapolation to time 0 (zero).
[C]: Value after 72 hours off hydrolysis.
[D]: Values for amino acids other than Leu were calculated with the value for Leu as the basis.
[E]: trp cannot be determined because it is decomposed under the conditions of this analysis.
[F]: For GIF146, values after 24 hours of hydrolysis are given.

(2) Analysis of carboxyl terminal

The above sample extracted from the SDS gel electrophoretic band was subjected to appropriate pretreatment (adjustment of pH, volume, etc.). Thereafter, a solution of carboxypeptidase A or a mixture of carboxypeptidase A and B in an appropriate concentration was added. After the subsequent 4 hours of reaction at 37°C, 0.02 N HCl was added, the pH and volume were further adjusted with 70% formic acid and then amino acid analysis was performed by the conventional method.

For the GIF135 sample treated with carboxypeptidase A and B, the amino acids detected were Ser (1.0), Arg (1.8), Lys (2.7), Gly (0.9), Thr (1.1) and Ala (1.0) alone (the numerical values given in the parentheses being based on the mole ratio values for the respective amino acids as obtained upon acid hydrolysis of the same GIF135 sample). For the sample treated with carboxypeptidase A, Ser (0.7), Arg (0.2) and Lys (0.1) were detected. The above results strongly suggest that the carboxyl terminal portion of GIF135 has the expected amino acid sequence.

Japanese Patent Applications Nos. 57-86180, 58-132524 and 58-162337 referred to herein correspond to European Patent Applications Nos. 83302915.0, 84304901.6 and 84305996.5 respectively.

Strains WA802 and W3110 are available from E. Coli Genetic Stock Control (USA).

**Claims**

1. A physiologically active polypeptide which has the amino acid sequence:

```
              1
     X   Cys Tyr Cys Gln Asp Pro Tyr Val Lys
             10
         Glu Ala Glu Asn Leu Lys Lys Tyr Phe
             20
         Asn Ala Gly His Ser Asp Val Ala Asp
                 30
         Asn Gly Thr Leu Phe Leu Gly Ile Leu
                     40
         Lys Asn Trp Lys Glu Glu Ser Asp Arg
                         50
         Lys Ile Met Gln Ser Gln Ile Val Ser
                             60
         Phe Tyr Phe Lys Leu Phe Lys Asn Phe
                                 70
         Lys Asp Asp Gln Ser Ile Gln Lys Ser
                                     80
         Val Glu Thr Ile Lys Glu Asp Met Asn
                                         90
         Val Lys Phe Phe Asn Ser Asn Lys Lys
         Lys Arg Asp Asp Phe Glu Lys Leu Thr
         100
         Asn Tyr Ser Val Thr Asp Leu Asn Val
             110
         Gln Arg Lys Ala Ile His Glu Leu Ile
                 120
         Gln Val Met Ala Glu Leu Ser Pro Ala
                     130                 135
         Ala Lys Thr Gly Lys Arg Lys Arg Ser
```

wherein X is a hydrogen atom or a methionyl group.

2. A DNA sequence which codes for a polypeptide claimed in Claim 1.

3. A DNA sequence as claimed in Claim 2, which is a product of chemical synthesis.

4. A DNA sequence as claimed in Claim 2 or Claim 3, which contains the DNA sequence:

```
ATG |TGC| TAC TGC CAG GAC CCA TAC GTG
TAC |ACG| ATG ACG GTC CTG GGT ATG CAC

AAG GAA GCT GAA AAC CTG AAG AAA TAC

TTC CTT CGA CTT TTG GAC TTC TTT ATG

TTC AAC GCT GGT CAT TCT GAC GTT GCT

AAG TTG CGA CCA GTA AGA CTG CAA CGA
```

GAC AAC GGT ACT CTG TTC CTG GGT ATC

CTG TTG CCA TGA GAC AAG GAC CCA TAG

CTG AAA AAC TGG AAA GAA GAA TCT GAC

GAC TTT TTG ACC TTT CTT CTT AGA CTG

TCT TTC TAC TTC AAG CTG TTC AAA AAC

AGA AAG ATG AAG TTC GAC AAG TTT TTG

TTC AAG GAC GAC CAG TCT ATC CAG AAA

AAG TTC CTG CTG GTC AGA TAG GTC TTT

TCT GTT GAA ACT ATC AAG GAA GAC ATG

AGA CAA CTT TGA TAG TTC CTT CTG TAC

AAC GTT AAG TTC TTC AAC TCT AAC AAG

TTG CAA TTC AAG AAG TTG AGA TTG TTC

AAA AAG CGT GAC GAC TTC GAA AAG CTT

TTT TTC GCA CTG CTG AAG CTT TTC GAA

ACT AAC TAC TCT GTT ACT GAC CTT AAT

TGA TTG ATG AGA CAA TGA CTG GAA TTA

GTA CAG CGT AAA GCT ATC CAT GAA CTG

CAT GTC GCA TTT CGA TAC GTA CTT GAC

ATC CAG GTT ATG GCT GAA CTG TCC CCG

TAG GTC CAA TAC CGA CTT GAC AGG GGC

GCT GCT AAA ACT GGT AAG CGT AAA AGA

CGA CGA TTT TGA CCA TTC GCA TTT TCT

TCT

AGA

wherein the sequence in the square ▢ may

alternatively be $\begin{matrix} TGT \\ ACA \end{matrix}$ .

5. A DNA sequence as claimed in any one of Claims 2 to 4, wherein the DNA sequence comprises a DNA fragment containing an *Eco*RI cleavage site connected upstream from said DNA sequence and a DNA fragment containing one or two termination condons (TAA or TAG) and a *Sa*/I cleavage site connected downstream from said DNA sequence.

6. An expression vector in which the DNA sequence coding for a polypeptide claimed in Claim 1 is effectively connected with a DNA sequence capable of causing expression of said polypeptide.

7. An expression vector as claimed in Claim 6 which contains the *tac* promoter, which is a hybrid promoter between the *Escherichia coli trp* and *lac* promoters, the lactose repressor gene *laci*[q] and a plasmid DNA having the function of increasing the plasmid copy number.

8. An expression vector as claimed in Claim 6 which has a modification of the untranslated 5'-end region of the *Escherichia coli* lipoprotein-regulating structural gene.

9. An expression vector as claimed in Claim 7 which is pYtacGIF135 or ptacGIF135.

10. An expression vector as claimed in Claim 7 which is pINGIF135.

11. A single cell microorganism transformant obtained by transformation with an expression vector with a gene coding for a polypeptide claimed in Claim 1 inserted therein a manner such that said gene can be expressed.

12. A transformant as claimed in Claim 11, wherein said expression vector is the expression vector as claimed in any one of the Claims 6 to 10.

13. A transformant as claimed in Claim 12, wherein the microorganism transformed is a strain of *Escherichia coli*.

14. A transformant as claimed in Claim 13 which is the strain WA802/pYtacGIF135 or WA802/ptacGIF135.

15. A transformant as claimed in Claim 13 which is the strain WA802/pINGIF135 or W3110/pINGIF135.

16. A method of producing a polypeptide as claimed in Claim 1, which comprises cultivating the transformant as claimed in any one of Claims 8 to 11 and harvesting said polypeptide.

17. A culture of a transformant as claimed in any one of claims 11 to 15, which contains a polypeptide as claimed in Claim 1.

18. A polypeptide as claimed in Claim 1 for use as an antiviral, antitumor or immunoregulating agent in the treatment of the human or animal body.

19. An antiviral, antitumor or immunoregulating composition comprising a polypeptide as claimed in Claim 1 as an antiviral, antitumor or immunoregulating agent.

20. The use of a polypeptide as claimed in Claim 1 in the manufacture of a medicament for use in the treatment of the human or animal body.

**Patentansprüche**

1. Ein physiologisch aktives Polypeptid welches die Aminosäuresequenz:

```
X  Cys Tyr Cys Gln Asp Pro Tyr Val Lys
   1
   10
   Glu Ala Glu Asn Leu Lys Lys Tyr Phe
       20
   Asn Ala Gly His Ser Asp Val Ala Asp
           30
   Asn Gly Thr Leu Phe Leu Gly Ile Leu
               40
   Lys Asn Trp Lys Glu Glu Ser Asp Arg
                   50
   Lys Ile Met Gln Ser Gln Ile Val Ser
                       60
   Phe Tyr Phe Lys Leu Phe Lys Asn Phe
                           70
   Lys Asp Asp Gln Ser Ile Gln Lys Ser
                               80
   Val Glu Thr Ile Lys Glu Asp Met Asn
                                   90
   Val Lys Phe Phe Asn Ser Asn Lys Lys
   Lys Arg Asp Asp Phe Glu Lys Leu Thr
   100
   Asn Tyr Ser Val Thr Asp Leu Asn Val
       110
   Gln Arg Lys Ala Ile His Glu Leu Ile
           120
   Gln Val Met Ala Glu Leu Ser Pro Ala
               130                 135
   Ala Lys Thr Gly Lys Arg Lys Arg Ser
```

aufweist, in der X ein Wasserstoffatom oder eine Methionylgruppe ist.

2. Eine DNA-Sequenz, die für das Polypeptid gemäß Anspruch 1 kodiert.

3. Eine DNA-Sequenz nach Anspruch 2, dadurch gekennzeichnet, daß sie ein chemisches Syntheseprodukt ist.

4. Eine DNA-Sequenz nach den Ansprüchen 2 oder 3, dadurch gekennzeichnet, daß sie die DNA-Sequenz

```
ATG TGC TAC TGC CAG GAC CCA TAC GTG

TAC ACG ATG ACG GTC CTG GGT ATG CAC

AAG GAA GCT GAA AAC CTG AAG AAA TAC

TTC CTT CGA CTT TTG GAC TTC TTT ATG

TTC AAC GCT GGT CAT TCT GAC GTT GCT

AAG TTG CGA CCA GTA AGA CTG CAA CGA

GAC AAC GGT ACT CTG TTC CTG GGT ATC

CTG TTG CCA TGA GAC AAG GAC CCA TAG

CTG AAA AAC TGG AAA GAA GAA TCT GAC

GAC TTT TTG ACC TTT CTT CTT AGA CTG

TCT TTC TAC TTC AAG CTG TTC AAA AAC

AGA AAG ATG AAG TTC GAC AAG TTT TTG

TTC AAG GAC GAC CAG TCT ATC CAG AAA

AAG TTC CTG CTG GTC AGA TAG GTC TTT

TCT GTT GAA ACT ATC AAG GAA GAC ATG

AGA CAA CTT TGA TAG TTC CTT CTG TAC

AAC GTT AAG TTC TTC AAC TCT AAC AAG

TTG CAA TTC AAG AAG TTG AGA TTG TTC

AAA AAG CGT GAC GAC TTC GAA AAG CTT

TTT TTC GCA CTG CTG AAG CTT TTC GAA

ACT AAC TAC TCT GTT ACT GAC CTT AAT

TGA TTG ATG AGA CAA TGA CTG GAA TTA

GTA CAG CGT AAA GCT ATC CAT GAA CTG

CAT GTC GCA TTT CGA TAC GTA CTT GAC

ATC CAG GTT ATG GCT GAA CTG TCC CCG

TAG GTC CAA TAC CGA CTT GAC AGG GGC

GCT GCT AAA ACT GGT AAG CGT AAA AGA

CGA CGA TTT TGA CCA TTC GCA TTT TCT
```

TCT

AGA

enthält, in der die Sequenz in dem Quadrat [ ]

alternativ $\frac{TGT}{ACA}$ sein kann.

5. Eine DNA-Sequenz nach einem der vorangehenden Ansprüche 2 bis 4, dadurch gekennzeichnet, daß die DNA-Sequenz ein DNA-Fragment enthält, welches ein n *EcoRI*-Schnittstelle besitzt, die oberhalb der genannten DNA-Sequenz angeordnet ist und ein DNA-Fragment, das ein oder zwei Terminationskodons (TAA oder TAG) und eine *SalI*-Schnittstelle besitzt, die unterhalb der DNA-Sequenz angeordnet ist.

6. Ein Expressionsvector, innerhalb dessen die für das Polypeptid gemäß Anspruch 1 kodierende DNA-Sequenz wirksam mit einer DNA-Sequenz verbunden ist, welche in der Lage ist, die Expression des Polypeptids zu bewirken.

7. Ein Expressionsvector gemäß Anspruch 6, welcher den tac-Promoter, der ein Hybridpromotor aus den *Escherichia coli trp* und *lac* Promotoren ist, das Lactose Repressorgen *laci*[n] und eine Plasmid DNA aufweist, die die Funktion hat, die Zahl der Plasmidkopien zu erhöhen.

8. Ein Expressionsvector gemäß Anspruch 6, welcher eine Modifikation der nicht-transkribierten Region am 5'-Ende des *Escherichia coli* Lipoprotein-regulierenden Strukturgens aufweist.

9. Ein Expressionsvector gemäß Anspruch 7, dadurch gekennzeichnet, daß er pYtacGIF135 oder ptacGIF135 ist.

10. Ein Expressionsvector nach Anspruch 7, dadurch gekennzeichnet, daß er pINGIF135 ist.

11. Ein transformierter einzelliger Mikroorganismus, erhalten durch Transformation mit einem Expressionsvector, in den ein für das Polypeptid gemäß Anspruch 1 kodierendes Gen so insertiert ist, daß das Gen exprimiert werden kann.

12. Ein Transformant gemäß Anspruch 11, dadurch gekennzeichnet, daß der Expressionsvector der Expressionsvector nach einem der Ansprüche 6 bis 10 ist.

13. Ein Transformant gemäß Anspruch 12, dadurch gekennzeichnet, daß der transformierte Mikroorganismus ein Stamm von *Escherichia coli* ist.

14. Ein Transformant gemäß Anspruch 13, dadurch gekennzeichnet, daß er der Stamm WA802/pYtacGIF135 oder WA802/ptacGIF135 ist.

15. Ein Transformant gemäß Anspruch 13, dadurch gekennzeichnet, daß es der Stamm WA802/pINGIF135 oder W3110/pINGIF135 ist.

16. Verfahren zum Herstellen eines Polypeptids gemäß Anspruch 1, dadurch gekennzeichnet, daß man den Transformanten gemäß jeglichem der Ansprüche 8 bis 11 kultiviert und das Polypeptid erntet.

17. Eine Kultur eines Transformanten gemäß jeglichem der Ansprüche 11 bis 15, dadurch gekennzeichnet, daß sie das Polypeptid gemäß Anspruch 1 enthält.

18. Eine Polypeptid gemäß Anspruch 1 zur Verwendung als antivirales, antitumor oder immunregulierendes Mittel bei der Behandlung des menschlichen oder tierischen Körpers.

19. Antivirale, antitumor oder immunregulierende Zusammensetzung mit einem Gehalt an einem Polypeptid gemäß Anspruch 1 als einem antiviralen, antitumor oder immunregulierenden Mittel.

20. Verwendung eines Polypeptids gemäß Anspruch 1 bei der Herstellung eines Arzneimittels zur Verwendung bei der Behandlung des menschlichen oder tierischen Körpers.

**Revendications**

1. Polypeptide physiologiquement actif qui a la séquence d'amino-acides:

```
        1
X     Cys Tyr Cys Gln Asp Pro Tyr Val Lys
      10
      Glu Ala Glu Asn Leu Lys Lys Tyr Phe
            20
      Asn Ala Gly His Ser Asp Val Ala Asp
                30
      Asn Gly Thr Leu Phe Leu Gly Ile Leu
                    40
      Lys Asn Trp Lys Glu Glu Ser Asp Arg
                        50
      Lys Ile Met Gln Ser Gln Ile Val Ser
                            60
      Phe Tyr Phe Lys Leu Phe Lys Asn Phe
                                70
      Lys Asp Asp Gln Ser Ile Gln Lys Ser
                                    80
      Val Glu Thr Ile Lys Glu Asp Met Asn
                                        90
      Val Lys Phe Phe Asn Ser Asn Lys Lys

      Lys Arg Asp Asp Phe Glu Lys Leu Thr
      100
      Asn Tyr Ser Val Thr Asp Leu Asn Val
          110
      Gln Arg Lys Ala Ile His Glu Leu Ile
              120
      Gln Val Met Ala Glu Leu Ser Pro Ala
                  130                 135
      Ala Lys Thr Gly Lys Arg Lys Arg Ser
```

dans laquelle X est un atome d'hydrogène ou un groupement méthionyle.

2. Séquence d'ADN qui code pour un polypeptide selon la revendication 1.

3. Séquence d'ADN selon la revendication 2, qui est un produit de synthèse chimique.

4. Séquence d'ADN selon la revendication 2 ou la revendication 3, qui contient la séquence d'ADN:

```
ATG TGC TAC TGC CAG GAC CCA TAC GTG

TAC ACG ATG ACG GTC CTG GGT ATG CAC

AAG GAA GCT GAA AAC CTG AAG AAA TAC

TTC CTT CGA CTT TTG GAC TTC TTT ATG

TTC AAC GCT GGT CAT TCT GAC GTT GCT

AAG TTG CGA CCA GTA AGA CTG CAA CGA

GAC AAC GGT ACT CTG TTC CTG GGT ATC

CTG TTG CCA TGA GAC AAG GAC CCA TAG

CTG AAA AAC TGG AAA GAA GAA TCT GAC

GAC TTT TTG ACC TTT CTT CTT AGA CTG
```

```
TCT TTC TAC TTC AAG CTG TTC AAA AAC

AGA AAG ATG AAG TTC GAC AAG TTT TTG

TTC AAG GAC GAC CAG TCT ATC CAG AAA

AAG TTC CTG CTG GTC AGA TAG GTC TTT

TCT GTT GAA ACT ATC AAG GAA GAC ATG

AGA CAA CTT TGA TAG TTC CTT CTG TAC

AAC GTT AAG TTC TTC AAC TCT AAC AAG

TTG CAA TTC AAG AAG TTG AGA TTG TTC

AAA AAG CGT GAC GAC TTC GAA AAG CTT

TTT TTC GCA CTG CTG AAG CTT TTC GAA

ACT AAC TAC TCT GTT ACT GAC CTT AAT

TGA TTG ATG AGA CAA TGA CTG GAA TTA

GTA CAG CGT AAA GCT ATC CAT GAA CTG

CAT GTC GCA TTT CGA TAC GTA CTT GAC

ATC CAG GTT ATG GCT GAA CTG TCC CCG

TAG GTC CAA TAC CGA CTT GAC AGG GGC

GCT GCT AAA ACT GGT AAG CGT AAA AGA

CGA CGA TTT TGA CCA TTC GCA TTT TCT

TCT

AGA
```

où la séquence dans le carré. ☐ peut également

```
       TGT
être
       ACA
```

5. Séquence d'ADN selon l'une quelconque des revendications 2 à 4, dans laquelle la séquence d'ADN comprend un fragment d'ADN contenant un site de coupure *Eco*RI lié en amont de ladite séquence d'ADN, et un fragment d'ADN contenant 1 ou 2 codons d'arrêt (TAA ou TAG) et un site de coupure *Sal*I lié en aval de ladite séquence d'ADN.

6. Vecteur d'expression dans lequel la séquence d'ADN codant pour un polypeptide selon la revendication 1 est efficacement lié avec une séquence d'ADN pouvant provoquer l'expression dudit polypeptide.

7. Vecteur d'expression selon la revendication 6 qui contient le promoteur *tac*, qui est un promoteur hybride entre les promoteurs *trp* et *lac* de *Escherichia coli*, le gène répresseur de lactose *laci*ᴿ et un ADN plasmidique ayant la fonction d'augmenter le nombre de copies du plasmide.

8. Vecteur d'expression selon la revendication 6, qui a une modification de la région terminale 5' non traduite du gène de structure régulant la lipoprotéine de *Escherichia coli*.

9. Vecteur d'expression selon la revendication 7, qui est pYtacGIF135 ou ptacGIF135.

10. Vecteur d'expression selon la revendication 7 qui est pINGIF135.

11. Transformant de microorganisme unicellulaire obtenu par transformation avec un vecteur d'expression ayant un gène codant pour un polypeptide selon la revendication 1 qui y est inséré de telle manière que ledit gène puisse s'exprimer.

12. Transformant selon la revendication 11, dans lequel ledit vecteur d'expression est le vecteur d'expression selon l'une quelconque des revendications 6 à 10.

13. Transformant selon la revendication 12, dans lequel le microorganisme transformé est une souche d'*Escherichia coli*.

14. Transformant selon la revendication 13, qui est la souche WA802/pYtacGIF135 ou WA802/ptacGIF135.

15. Transformant selon la revendication 13, qui est la souche WA802/pINGIF135 ou W3110/pINGIF135.

16. Procédé de production d'un polypeptide selon la revendication 1, qui consiste à cultiver le transformant selon l'une quelconque des revendications 8 à 11 et à recueillir ledit polypeptide.

17. Culture d'un transformant selon les revendications 11 à 15, qui contient un polypeptide selon la revendication 1.

18. Polypeptide selon la revendication 1, utilisé comme agent antiviral, antitumoral ou immunorégulateur dans le traitement de l'organisme humain ou animal.

19. Composition antivirale, antitumorale ou immunorégulatrice comprenant un polypeptide selon la revendication 1, en tant qu'agent antiviral, antitumoral ou immunorégulateur.

20. Utilisation d'un polypeptide selon la revendication 1 dans la fabrication d'un médicament pour l'utilisation dans le traitement de l'organisme humain ou animal.

# FIG.1

Construction of pYtacGIF135

# FIG.2

# FIG.3

130 131 132 133 134 135 136
Gly Lys Arg Lys Arg Ser Gln
GGT AAA CGT AAA AGA TCT CAG — — — — — — CAG TAA GTC GAC
CCA TTT GCA TTT TCT AGA GTC                GTC ATT CAG CTG
                    Bgl II                           SalI

146
Gln stop

Bgl II - SalI digestion

5'
GATCTTAATAG
AATTATCAGCT
            3'
chemically synthesized
DNA fragment

130 131 132 133 134 135
Gly Lys Arg Lys Arg Serstopstop
GGT AAA CGT AAA AGA TCT TAA TAG TCG AC
CCA TTT GCA TTT TCT AGA ATT ATC AGC TG
                    Bgl II        SalI